# EUROPÄISCHE PATENTSCHRIFT

(11) **EP 0 775 704 B1**
(45) Veröffentlichungstag und Bekanntmachung des Hinweises auf die Patenterteilung: **06.06.2001**
(21) Anmeldenummer: 96118043.7
(22) Anmeldetag: 11.11.1996
(51) Int. Cl.: C07D 471/12, A61K 31/41

(54) **Kristallines Hydrochlorid von (R)-(-)-2-[N-[4-(1,1-Dioxido-3-oxo-2,3-dihydrobenzisothiazol-2-yl)-butyl]-aminomethyl]-chroman**
Crystalline hydrochloride of (R)-(-)-2-[N-[4-(1,1-Dioxido-3-oxo-2,3-dihydrobenzisothiazol-2-yl)-butyl]-aminomethyl]-chroman
Chlorhydrate cristallin du (R)-(-)-2-[N-[4-(1,1-Dioxido-3-oxo-2,3-dihydrobenzisothiazol-2-yl)-butyl]-aminométhyl]-chromane

(30) Priorität: 22.11.1995 DE 19543478
(43) Veröffentlichungstag der Anmeldung: 28.05.1997
(73) Patentinhaber: BAYER AG, 51368 Leverkusen (DE)
(72) Erfinder: Grunenberg, Alfons, Dr., 41539 Dormagen (DE); Brehm, Oliver, Dr., 42115 Wuppertal (DE); Conrad, Michael, Dr., 42327 Wuppertal (DE); Seidel, Dietrich, Dr., 42111 Wuppertal (DE)

(56) Entgegenhaltungen:
- EP-A- 0 352 613

## Beschreibung

Die Erfindung betrifft die kristalline Form des Hydrochlorides der Verbindung (R)-(-)-2- {N-[4-(1,1-Dioxido-3-oxo-2,3-dihydro-benzisothiazol-2-yl)-butyl]-amino-methyl}-chroman, Verfahren zur Herstellung sowie die Verwendung in Arzneimitteln insbesondere in Arzneimitteln mit neuroprotektiver Wirkung.

Das Hydrochlorid der Verbindung (R)-(-)-2-{N-[4-(1,1-Dioxido-3-oxo-2,3-dihydro-benzisothiazol-2-yl)-butyl]-aminomethyl}-chroman hat die Formel (I) 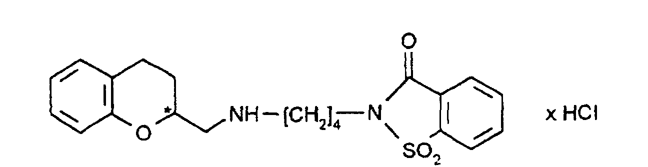 und wird im folgenden als DBCH bezeichnet. Die Verbindung der Formel (I) wird beispielsweise in der europäischen Patentschrift EP 352 613 als Beispiel 92 H offenbart und aufgrund ihrer Eigenschaften als 5-HT_{1A}-Agonist unter anderem als neuroprotektiver Wirkstoff in Arzneimitteln, insbesondere zur Behandlung und Prävention neuronaler Degenerationen infolge ischämischer Effekte wie Schlaganfall vorgeschlagen.

In der erwähnten Patentschrift wird auch die Herstellung von DBCH beschrieben. Sie erfolgt im allgemeinen durch Umsetzung des optisch aktiven 2-Aminomethylchromans mit N-4-Brombutylsaccharin in einem inerten Lösungsmittel in Anwesenheit einer Base. Auf diese Weise wird die Verbindung DBCH in einer kristallinen Modifikation (im folgenden Modifikation II genannt) erhalten. Der Schmelzpunkt wird mit 192-194 °C angegeben.

Es hat sich gezeigt, daß Modifikation II metastabil ist und sich bei hoher Feuchtigkeit (85 % relative Feuchte, Raumtemperatur) teilweise umwandelt. Sie ist aus diesem Grund nur eingeschränkt für die Verwendung in pharmazeutischen Formulierungen geeignet.

Überraschenderweise wurde nun eine kristalline Form des Hydrochlorid von (R)-(-)-2-{N-[4-(1,1-Dioxido-3-oxo-2,3-dihydro-benzisothiazol-2-yl)-butyl]-aminomethyl}chroman (nachfolgend Modifikation V genannt) gefunden, die diese Nachteile nicht besitzt. Diese kristalline Form des DBCH ist thermodynamisch stabil und wandelt sich selbst bei hoher Luftfeuchtigkeit (85 % relative Feuchte, Raumtemperatur) nicht um. Sie ist aus diesem Grund besonders gut geeignet für feste Arzneimittel-Formulierungen.

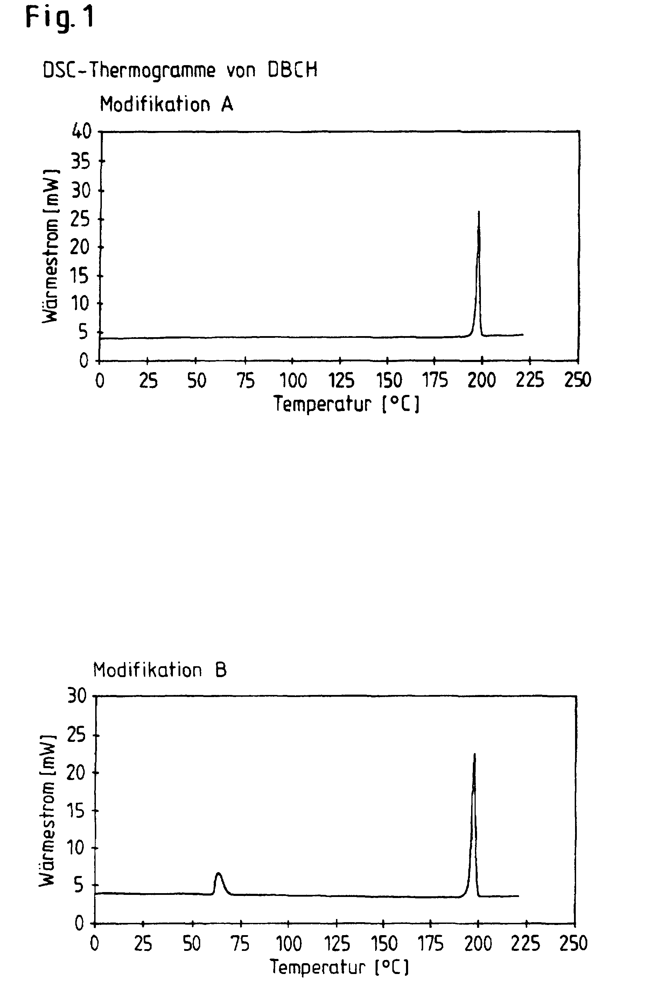

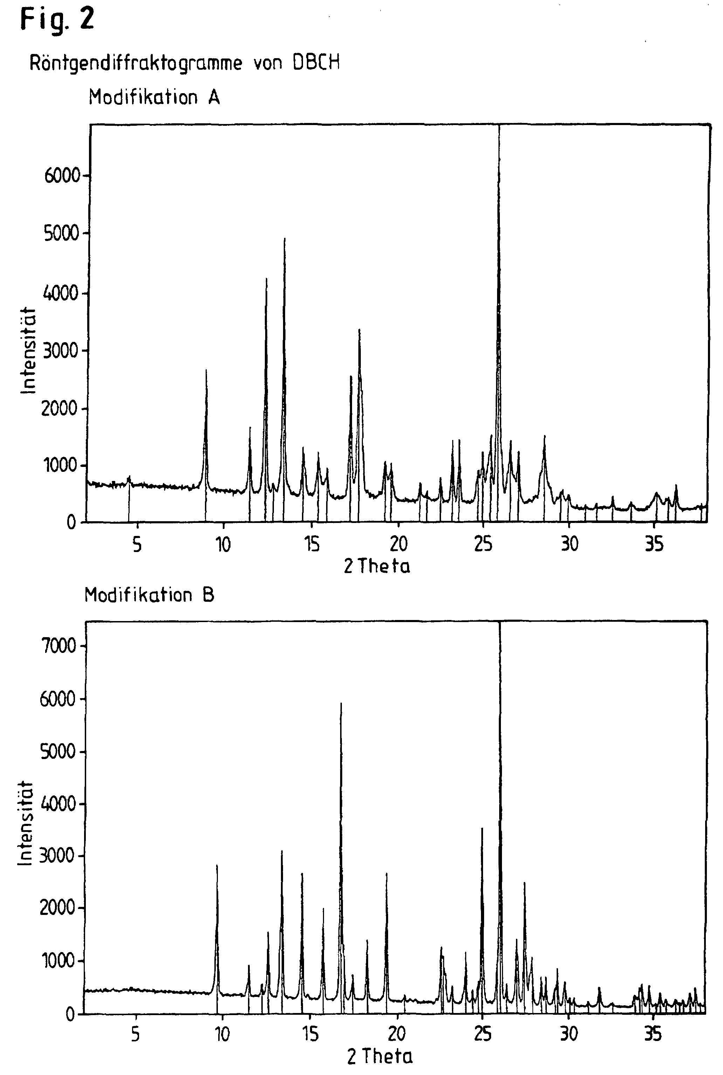

Die erfindungsgemäße kristalline Form (Modifikation V) des DBCH ist durch folgende physikochemische Parameter definiert:
1. Der Schmelzpunkt der erfindungsgemäßen kristallinen Form kann experimentell nicht bestimmt werden, da beim Aufheizen eine fest-fest-Phasenumwandlung bei ca. 60 °C zu Modifikation I stattfindet (endothermer Peak im DSC-Thermogramm, Fig. 1). Die Umwandlungswärme beträgt 24 J/g.
2. Röntgendiffraktogramm (s. Figur 2):

| Mod. V [2 Theta] | Mod. V [2 Theta] | Mod. V [2 Theta] | Mod. V [2 Theta] |
|---|---|---|---|
| 9,7 | 22,5 | 28,4 | 35,1 |
| 11,5 | 22,6 | 27,7 | 35,3 |
| 12,3 | 23,2 | 29,2 | 35,7 |
| 12,6 | 24,0 | 29,3 | 36,2 |
| 13,4 | 24,4 | 29,8 | 36,4 |
| 14,5 | 24,7 | 30,1 | 36,7 |
| 15,7 | 24,9 | 31,1 | 37,0 |
| 16,8 | 25,8 | 31,8 | 37,4 |
| 16,9 | 26,0 | 32,6 | 37,6 |
| 17,5 | 26,3 | 33,9 | |
| 18,3 | 26,9 | 34,1 | |
| 19,4 | 27,4 | 34,3 | |
| 20,4 | 27,9 | 34,7 | |

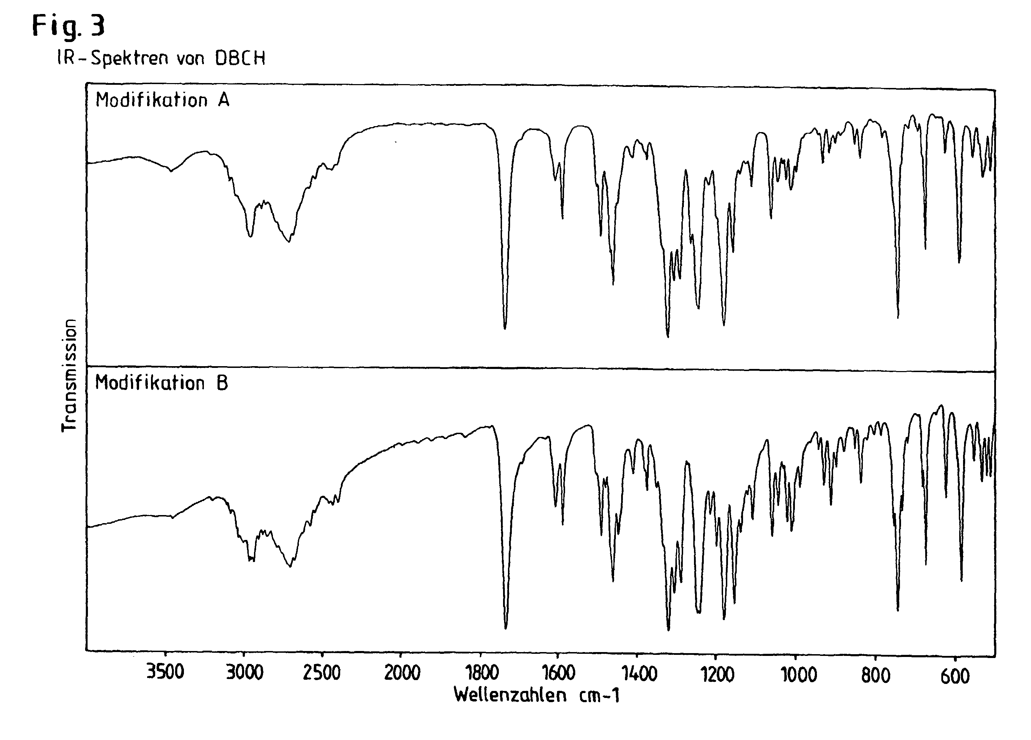

3. IR-Spektrum (Figur 3):

| Mod. V [cm⁻¹] | Mod. V [cm⁻¹] | Mod. V [cm⁻¹] | Mod. V [cm⁻¹] |
|---|---|---|---|
| 621 | 1010 | 1373 | 2847 |
| 672 | 1021 | 1408 | 2873 |
| 680 | 1044 | 1445 | 2899 |
| 716 | 1059 | 1458 | 2930 |
| 731 | 1106 | 1488 | 2949 |
| 743 | 1137 | 1585 | 2961 |
| 752 | 1153 | 1603 | 2993 |
| 783 | 1179 | 1690 | 3025 |
| 800 | 1197 | 1733 | 3073 |
| 835 | 1213 | 2389 | 3097 |
| 849 | 1242 | 2424 | 3189 |
| 876 | 1242 | 2448 | 3444 |
| 897 | 1248 | 2541 | |
| 910 | 1288 | 2571 | |
| 927 | 1305 | 2673 | |
| 940 | 1320 | 2701 | |
| 987 | 1348 | 2785 | |

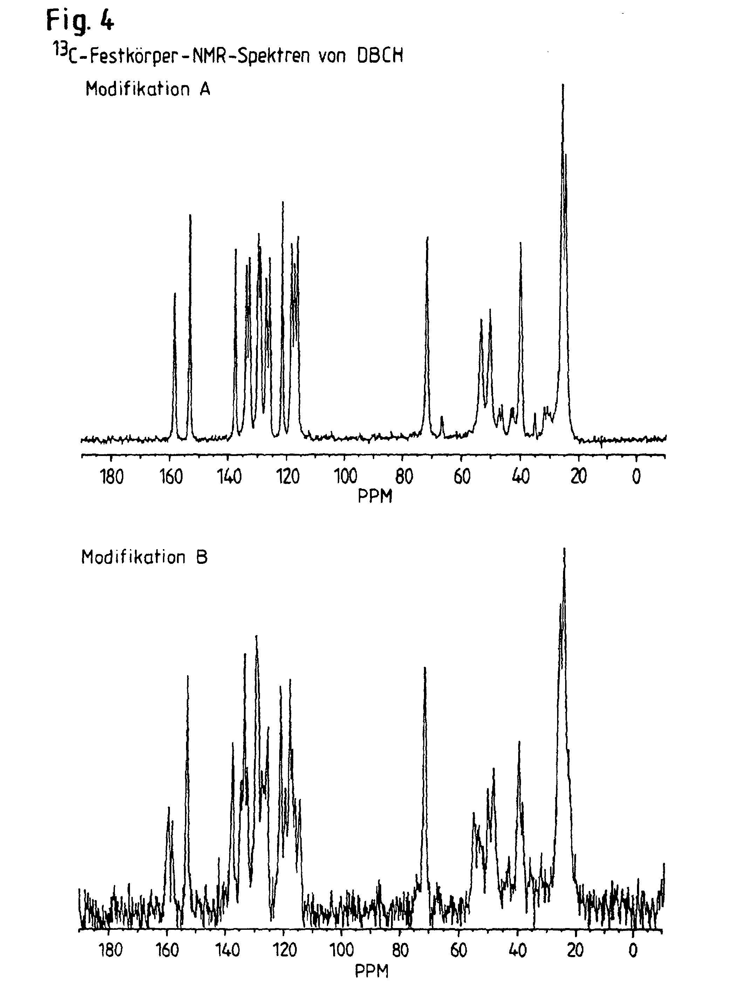

4. ¹³C-NMR-Festkörperspektrum (Figur 4)

| Mod. V [ppm] | Mod. V [ppm] | Mod. V [ppm] | Mod. V [ppm] |
|---|---|---|---|
| 159 | 128 | 72 | 26 |
| 158 | 126 | 55 | 25 |
| 153 | 121 | 54 | 23 |
| 137 | 120 | 50 | |
| 135 | 118 | 49 | |
| 134 | 117 | 40 | |
| 133 | 116 | 39 | |
| 130 | 115 | 27 | |

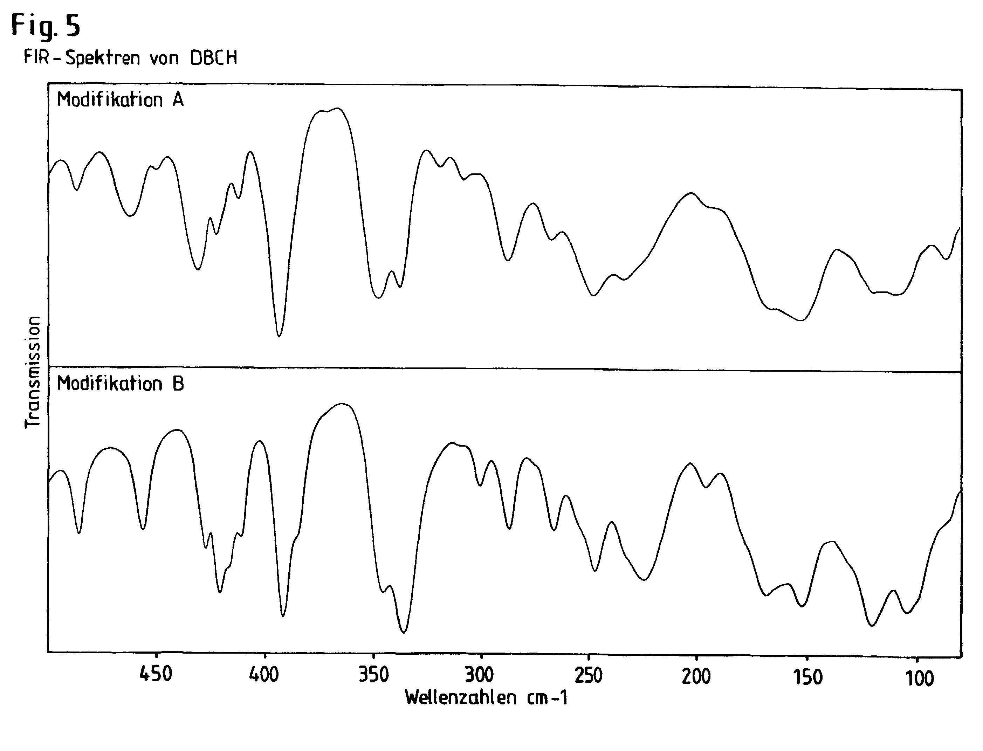

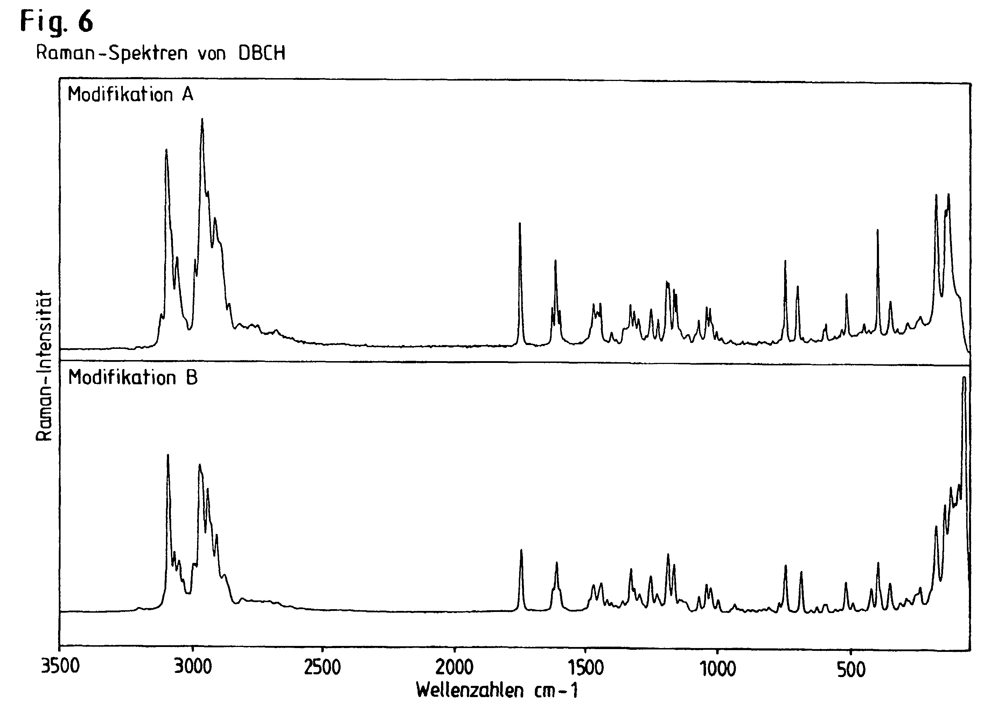

5. FIR-Spektrum (s. Figur 5) 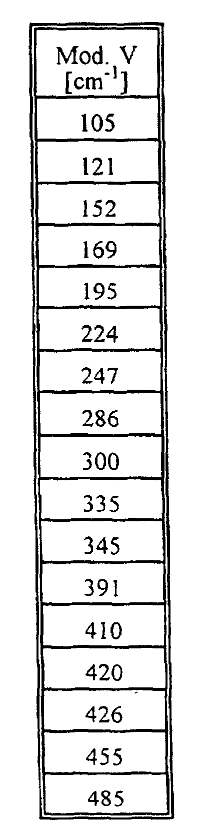
6. Raman-Spektrum (s Figur 6)

| Mod. V [cm⁻¹] | Mod. V [cm⁻¹] | Mod. V [cm⁻¹] | Mod. V [cm⁻¹] |
|---|---|---|---|
| 9 | 644 | 1379 | 3044 |
| 105 | 680 | 1394 | 3061 |
| 119 | 739 | 1411 | 3082 |
| 142 | 763 | 1433 | |
| 174 | 988 | 1462 | |
| 234 | 1016 | 1476 | |
| 286 | 1033 | 1600 | |
| 309 | 1062 | 1613 | |
| 348 | 1158 | 1736 | |
| 393 | 1181 | 2869 | |
| 419 | 1223 | 2900 | |
| 487 | 1245 | 2933 | |
| 513 | 1288 | 2963 | |
| 587 | 1307 | 2983 | |
| 595 | 1321 | 2991 | |
| 623 | 1351 | 3028 | |

Die Modifikation V ist in ihren physikochemischen Parametern (DSC-Schmelzverhalten, Röntgendiffraktogramm, IR-Spektrum, ¹³C-Festkörper-NMR-Spektrum, FIR-Spektrum und Raman-Spektrum (s. Figuren I bis 6)) deutlich von der Modifikation II zu unterscheiden. Der Schmelzpunkt der Modifiktion II beträgt 195 °C (DSC, Heizrate 10 K/Minute).

Die Herstellung der erfindungsgemäßen kristallinen Form (Modifikation V des DBCH) erfolgt im allgemeinen, indem man DBCH der Modifikation II in Wasser oder inerten organischen Stoffen, z.B. niederen Alkoholen, Ketonen oder Alkanen bis zum Erreichen des gewünschten Umwandlungsgrades, besonders bevorzugt bis zur quantitativen Umwandlung in Mod. V suspendiert. In der Regel findet diese Umwandlung bei Temperaturen von 0 °C bis 35 °C, bevorzugt bei 30 °C statt. Die erhaltenen Kristalle der Mod. V werden abgetrennt und zur Entfernung vorhandenen Lösungsmittels bei Raumtemperatur im Vakuum oder erhöhter Temperatur bis zur Gewichtskonstanz getrocknet.

Die erfindungsgemäße kristalline Modifikation V des DBCH kann in bekannter Weise in die üblichen Formulierungen überführt werden, wobei Formulierungen in denen der kristalline Wirkstoff in fester Form vorliegt, wie beispielsweise Tabletten, Dragees, Pillen, Granulataerosole, Suppositorien und Suspensionen geeignet sind. Der Wirkstoff wird hierbei unter Verwendung inerter nichttoxischer pharmazeutisch geeigneter Hilfs- und Trägerstoffe in die geeignete Formulierung überführt.

### Experimenteller Teil:

### Herstellung von Impfkristallen der Mod. V

0,5 g DBCH der Modifikation II werden in 8 ml Aceton:Methanol (7:1) suspendiert und 5 Tage bei Raumtemperatur gerührt. Der Rückstand wird abfiltriert und bei Raumtemperatur im Vakuum bis zur Gewichtskonstanz getrocknet. Zur Prüfung auf quantitative Umwandlung wird das IR-Spektrum aufgenommen.

### Herstellung Mod. II

Der Wirkstoff in Form der Mod. II wird bei der Wirkstoffsynthese durch Zugabe von ca. 1,5 mol äquivalenten Salzsäure erhalten. Mod. II wird aus Ethanol bei ca. 5-50 °C gefällt, abfiltriert, anschließend mit Ethanol gewaschen und unter Vakuum bei 95-100 °C getrocknet.

### Beispiele

### Beispiel 1

0,5 g DBCH der Modifikation II werden in 7 ml Aceton:Ethanol (6:1) suspendiert. Die Suspension wird mit der Modifikation V angeimpft und 5 Tage bei 0 °C gerührt. Der Rückstand wird abfiltriert und bei Raumtemperatur im Vakuum bis zur Gewichtskonstanz getrocknet Zur Prüfung auf quantitative Umwandlung wird das DSC-Thermogramm aufgenommen.

### Beispiel 2

0,5 g DBCH der Modifikation II werden in 6 ml Aceton:Ethanol (2:1) suspendiert. Die Suspension wird mit der Modifikation V angeimpft und 5 Tage bei Raumtemperatur gerührt. Der Rückstand wird abfiltriert und bei Raumtemperatur im Vakuum bis zur Gewichtskonstanz getrocknet. Zur Prüfung auf quantitative Umwandlung wird das IR-Spektrum aufgenommen.

### Beispiel 3

1,7 g DBCH der Modifikation II werden in 14 ml Aceton:Ethanol (6:1) suspendiert. Die Suspension wird mit der Modifikation V angeimpft und 6 Tage bei Raumtemperatur gerührt. Der Rückstand wird abfiltriert und bei Raumtemperatur im Vakuum bis zur Gewichtskonstanz getrocknet. Zur Prüfung auf quantitative Umwandlung wird das Röntgendiffraktogramm aufgenommen.

### Beispiel 4

0,4 g DBCH der Modifikation II werden in 7 ml Aceton:Ethanol (6:1) suspendiert. Die Suspension wird mit der Modifikation V angeimpft und eine Woche bei 30°C gerührt. Der Rückstand wird abfiltriert und bei Raumtemperatur im Vakuum bis zur Gewichtskonstanz getrocknet. Zur Prüfung auf quantitative Umwandlung wird das DSC-Thermogramm aufgenommen.

### Beispiel 5

0,5 g DBCH der Modifikation II werden in 10 ml iso-Propanol suspendiert. Die Suspension wird mit der Modifikation V angeimpft und 6 h bei Raumtemperatur gerührt. Der Rückstand wird abfiltriert und bei Raumtemperatur im Vakuum bis zur Gewichtskonstanz getrocknet. Zur Prüfung auf quantitative Umwandlung wird das DSC-Thermogramm aufgenommen.

## Patentansprüche

1. Kristalline Modifikation V von (R)-(-)-2-{N-[4-(1,1-Dioxido-3-oxo-2,3-di-hydro-benzisothiazol-2-yl)-butyl]-aminomethyl}-chroman-hydrochlorid, charakterisiert durch folgende Röntgendiffraktogramm-Daten.
| Mod. V [2 Theta] | Mod. V [2 Theta] | Mod. V [2 Theta] | Mod. V [2 Theta] |
|---|---|---|---|
| 9,7 | 22,5 , | 28,4 | 35,1 |
| 11,5 | 22,6 | 27,7 | 35,3 |
| 12,3 | 23,2 | 29,2 | 35,7 |
| 12,6 | 24,0 | 29,3 | 36,2 |
| 13,4 | 24,4 | 29,8 | 36,4 |
| 14,5 | 24,7 | 30,1 | 36,7 |
| 15,7 | 24,9 | 31,1 | 37,0 |
| 16,8 | 25,8 | 31,8 | 37,4 |
| 16,9 | 26,0 | 32,6 | 37,6 |
| 17,5 | 26,3 | 33,9 | |
| 18,3 | 26,9 | 34,1 | |
| 19,4 | 27,4 | 34,3 | |
| 20,4 | 27,9 | 34,7 | |

2. Kristallines Hydrochlorid nach Anspruch 1, das sich bei ca. 60 °C mit einer Umwandlungswärme von 24 J/g umwandelt.

3. Kristallines Hydrochlorid nach Anspruch 1 und 2 charakterisiert durch folgende IR-Spektral-Daten:
| Mod. V [cm⁻¹] | Mod. V [cm⁻¹] | Mod. V [cm⁻¹] | Mod. V [cm⁻¹] |
|---|---|---|---|
| 621 | 1010 | 1373 | 2847 |
| 672 | 1021 | 1408 | 2873 |
| 680 | 1044 | 1445 | 2899 |
| 716 | 1059 | 1458 | 2930 |
| 731 | 1106 | 1488 | 2949 |
| 743 | 1137 | 1585 | 2961 |
| 752 | 1153 | 1603 | 2993 |
| 783 | 1179 | 1690 | 3025 |
| 800 | 1197 | 1733 | 3073 |
| 835 | 1213 | 2389 | 3097 |
| 849 | 1242 | 2424 | 3189 |
| 876 | 1242 | 2448 | 3444 |
| 897 | 1248 | 2541 | |
| 910 | 1288 | 2571 | |
| 927 | 1305 | 2673 | |
| 940 | 1320 | 2701 | |
| 987 | 1348 | 2785 | |

4. Kristallines Hydrochlorid nach Anspruch 1 bis 3 charakterisiert durch die folgenden ¹³C-NMR-Festkörperspektral-Daten:
| Mod. V [ppm] | Mod. V [ppm] | Mod. V [ppm] | Mod. V [ppm] |
|---|---|---|---|
| 159 | 128 | 72 | 26 |
| 158 | 126 | 55 | 25 |
| 153 | 121 | 54 | 23 |
| 137 | 120 | 50 | |
| 135 | 118 | 49 | |
| 134 | 117 | 40 | |
| 133 | 116 | 39 | |
| 130 | 115 | 27 | |

5. Kristallines Hydrochlorid nach Anspruch 1 bis 4 charakterisiert durch folgende FIR-Spektral-Daten:

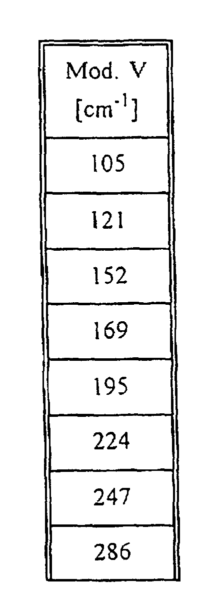

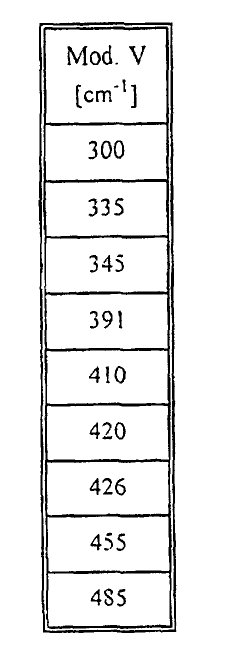

6. Kristallines Hydrochlorid nach Anspruch 1 bis 5 charakterisiert durch folgende Raman-Spektral-Daten:
| Mod. V [cm⁻¹] | Mod. V [cm⁻¹] | Mod. V [cm⁻¹] | Mod. V [cm⁻¹] |
|---|---|---|---|
| 9 | 644 | 1379 | 3044 |
| 105 | 680 | 1394 | 3061 |
| 119 | 739 | 1411 | 3082 |
| 142 | 763 | 1433 | |
| 174 | 988 | 1462 | |
| 234 | 1016 | 1476 | |
| 286 | 1033 | 1600 | |
| 309 | 1062 | 1613 | |
| 348 | 1158 | 1736 | |
| 393 | 1181 | 2869 | |
| 419 | 1223 | 2900 | |
| 487 | 1245 | 2933 | |
| 513 | 1288 | 2963 | |
| Mod. V [cm⁻¹] | Mod. V [cm⁻¹] | Mod. V [cm⁻¹] | Mod. V [cm⁻¹] |
|---|---|---|---|
| 587 | 1307 | 2983 | |
| 595 | 1321 | 2991 | |
| 623 | 1351 | 3028 | |

7. Verfahren zur Herstellung von kristallinem Hydrochlorid nach Anspruch 6, dadurch gekennzeichnet, daß man DBCH der Modifikation II in Wasser oder inerten organischen Stoffen suspendiert und quantitativ in Mod. V gemäß Anspruch 1 bis 6 überführt.

8. Arzneimittel enthaltend das kristalline Hydrochlorid nach Anspruch 1 bis 6.

9. Verwendung des kristallinen Hydrochlorides nach Anspruch 1 bis 5 zur Herstellung von Arzneimitteln.

## Claims

1. Crystalline modification V of (R)-(-)-2-{N-[4-(1,1-dioxido-3-oxo-2,3-dihydrobenzisothiazol-2-yl)-butyl]-aminomethyl}-chroman hydrochloride, characterized by the following X-ray diffraction data:
| Mod. V [2 Theta] | Mod. V [2 Theta] | Mod. V [2 Theta] | Mod. V [2 Theta] |
|---|---|---|---|
| 9.7 | 22.5 | 28.4 | 35.1 |
| 11.5 | 22.6 | 27.7 | 35.3 |
| 12.3 | 23.2 | 29.2 | 35.7 |
| 12.6 | 24.0 | 29.3 | 36.2 |
| 13.4 | 24.4 | 29.8 | 36.4 |
| 14.5 | 24.7 | 30.1 | 36.7 |
| 15.7 | 24.9 | 31.1 | 37.0 |
| 16.8 | 25.8 | 31.8 | 37.4 |
| 16.9 | 26.0 | 32.6 | 37.6 |
| 17.5 | 26.3 | 33.9 | |
| 18.3 | 26.9 | 34.1 | |
| 19.4 | 27.4 | 34.3 | |
| 20.4 | 27.9 | 34.7 | |

2. Crystalline hydrochloride according to Claim 1, which undergoes transformation at about 60°C with a heat of transformation of 24 J/g.

3. Crystalline hydrochloride according to any of Claims 1 and 2, characterized by the following IR spectral data:
| Mod. V [cm⁻¹] | Mod. V [cm⁻¹] | Mod. V [cm⁻¹] | Mod. V [cm⁻¹] |
|---|---|---|---|
| 621 | 1010 | 1373 | 2847 |
| 672 | 1021 | 1408 | 2873 |
| 680 | 1044 | 1445 | 2899 |
| 716 | 1059 | 1458 | 2930 |
| 731 | 1106 | 1488 | 2949 |
| 743 | 1137 | 1585 | 2961 |
| 752 | 1153 | 1603 | 2993 |
| 783 | 1179 | 1690 | 3025 |
| 800 | 1197 | 1733 | 3073 |
| 835 | 1213 | 2389 | 3097 |
| 849 | 1242 | 2424 | 3189 |
| 876 | 1242 | 2448 | 3444 |
| 897 | 1248 | 2541 | |
| 910 | 1288 | 2571 | |
| 927 | 1305 | 2673 | |
| 940 | 1320 | 2701 | |
| 987 | 1348 | 2785 | |

4. Crystalline hydrochloride according to any of Claims 1 to 3, characterized by the following solid-state ¹³C-NMR spectral data:
| Mod. V [ppm] | Mod.V [ppm] | Mod. V [ppm] | Mod.V [ppm] |
|---|---|---|---|
| 159 | 128 | 72 | 26 |
| 158 | 126 | 55 | 25 |
| 153 | 121 | 54 | 23 |
| 137 | 120 | 50 | |
| 135 | 118 | 49 | |
| 134 | 117 | 40 | |
| 133 | 116 | 39 | |
| 130 | 115 | 27 | |

5. Crystalline hydrochloride according to any of Claims 1 to 4, characterized by the following FIR spectral data:

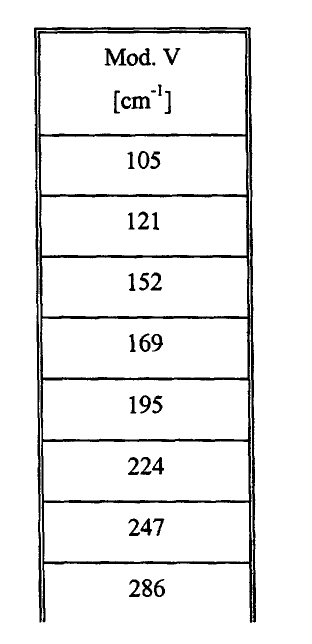

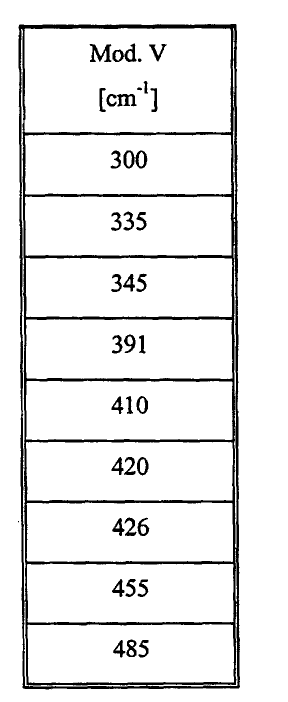

6. Crystalline hydrochloride according to any of Claims 1 to 5, characterized by the following Raman spectral data:
| Mod. V [cm⁻¹] | Mod. V [cm⁻¹] | Mod. V [cm⁻¹] | Mod. V [cm⁻¹] |
|---|---|---|---|
| 9 | 644 | 1379 | 3044 |
| 105 | 680 | 1394 | 3061 |
| 119 | 739 | 1411 | 3082 |
| 142 | 763 | 1433 | |
| 174 | 988 | 1462 | |
| 234 | 1016 | 1476 | |
| 286 | 1033 | 1600 | |
| 309 | 1062 | 1613 | |
| 348 | 1158 | 1736 | |
| Mod. V [cm⁻¹] | Mod. V [cm⁻¹] | Mod. V [cm⁻¹] | Mod. V [cm⁻¹] |
|---|---|---|---|
| 393 | 1181 | 2869 | |
| 419 | 1223 | 2900 | |
| 487 | 1245 | 2933 | |
| 513 | 1288 | 2963 | |
| 587 | 1307 | 2983 | |
| 595 | 1321 | 2991 | |
| 623 | 1351 | 3028 | |

7. Process for the preparation of crystalline hydrochloride according to Claim 6, characterized in that DBCH of modification II is suspended in water or inert organic substances and quantitatively converted into modification V according to any of Claims 1 to 6.

8. Medicament containing the crystalline hydrochloride according to any of Claims 1 to 6.

9. Use of the crystalline hydrochloride according to any of Claims 1 to 5 for the preparation of medicaments.

## Revendications

1. Modification cristalline V du chlorhydrate de (R) - (-) -2-{N-[4-(1,1-dioxydo-3-oxo-2,3-dihydrobenzisothiazole-2-yl)-butyl]-aminométhyl}-chromane, caractérisée par les valeurs suivantes dans le difractogramme des rayons X :
| Mod. V [2 Theta] | Mod. V [2 Theta] | Mod. V [2 Theta] | Mod. V [2 Theta] |
|---|---|---|---|
| 9,7 | 22,5 | 28,4 | 35,1 |
| 11,5 | 22,6 | 27,7 | 35,3 |
| 12,3 | 23,2 | 29,2 | 35,7 |
| 12,6 | 24,0 | 29,3 | 36,2 |
| 13,4 | 24,4 | 29,8 | 36,4 |
| 14,5 | 24,7 | 30,1 | 36,7 |
| 15,7 | 24,9 | 31,1 | 37,0 |
| 16,8 | 25,8 | 31,8 | 37,4 |
| 16,9 | 26,0 | 32,6 | 37,6 |
| 17,5 | 26,3 | 33,9 | |
| 18,3 | 26,9 | 34,1 | |
| 19,4 | 27,4 | 34,3 | |
| 20,4 | 27,9 | 34,7 | |

2. Chlorhydrate cristallin suivant la revendication 1, qui se transforme à environ 60°C avec une chaleur de transformation de 24 J/g.

3. Chlorhydrate cristallin suivant les revendications 1 et 2, caractérisé par les valeurs spectrales infrarouges suivantes :
| Mod. V [cm⁻¹] | Mod. V [cm⁻¹] | Mod. V [cm⁻¹] | Mod. V [cm⁻¹] |
|---|---|---|---|
| 621 | 1010 | 1373 | 2847 |
| 672 | 1021 | 1408 | 2873 |
| 680 | 1044 | 1445 | 2899 |
| 716 | 1059 | 1458 | 2930 |
| 731 | 1106 | 1488 | 2949 |
| 743 | 1137 | 1585 | 2961 |
| 752 | 1153 | 1603 | 2993 |
| 783 | 1179 | 1690 | 3025 |
| 800 | 1197 | 1733 | 3073 |
| 835 | 1213 | 2389 | 3097 |
| 849 | 1242 | 2424 | 3189 |
| 876 | 1242 | 2448 | 3444 |
| 897 | 1248 | 2541 | |
| 910 | 1288 | 2571 | |
| 927 | 1305 | 2673 | |
| 940 | 1320 | 2701 | |
| 987 | 1348 | 2785 | |

4. Chlorhydrate cristallin suivant les revendications 1 à 3, caractérisé par les valeurs suivantes du spectre de ¹³C-RMN sur corps solide :
| Mod. V [ppm] | Mod. V [ppm] | Mod. V (ppm] | Mod. V [ppm] |
|---|---|---|---|
| 159 | 128 | 72 | 26 |
| 158 | 126 | 55 | 25 |
| 153 | 121 | 54 | 23 |
| 137 | 120 | 50 | |
| 135 | 118 | 49 | |
| 134 | 117 | 40 | |
| 133 | 116 | 39 | |
| 130 | 115 | 27 | |

5. Chlorhydrate cristallin suivant les revendications 1 à 4, caractérisé par les valeurs suivantes du spectre d'infrarouge lointain :

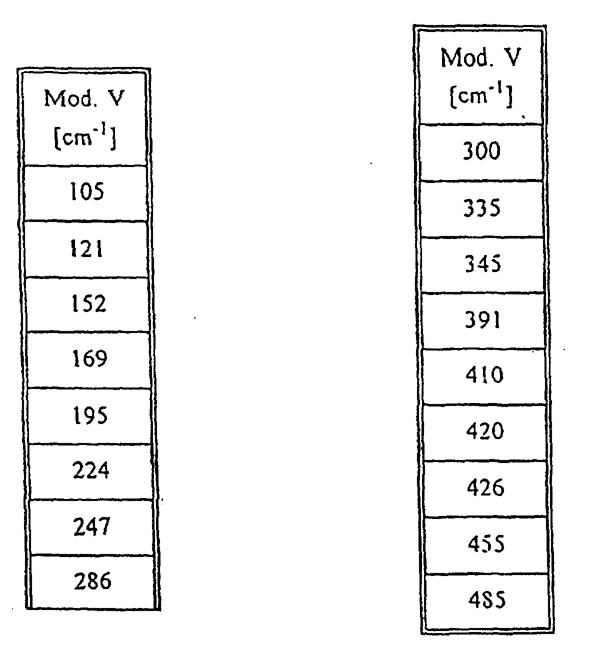

6. Chlorhydrate cristallin suivant les revendications 1 à 5, caractérisé par les caractéristiques de spectre Raman suivantes :
| Mod. V [cm⁻¹] | Mod. V [cm⁻¹] | Mod. V [cm⁻¹] | Mod. V [cm⁻¹] |
|---|---|---|---|
| 9 | 644 | 1379 | 3044 |
| 105 | 680 | 1394 | 3061 |
| 119 | 739 | 1411 | 3082 |
| 142 | 763 | 1433 | |
| 174 | 988 | 1462 | |
| 234 | 1016 | 1476 | |
| 286 | 1033 | 1600 | |
| 309 | 1062 | 1613 | |
| 348 | 1158 | 1736 | |
| 393 | 1181 | 2869 | |
| 419 | 1223 | 2900 | |
| 487 | 1245 | 2933 | |
| 513 | 1288 | 2963 | |
| Mod. V [cm⁻¹] | Mod. V [cm⁻¹] | Mod. V [cm⁻¹] | Mod. V [cm⁻¹] |
|---|---|---|---|
| 587 | 1307 | 2983 | |
| 595 | 1321 | 2991 | |
| 623 | 1351 | 3028 | |

7. Procédé de production du chlorhydrate cristallin suivant la revendication 6, caractérisé en ce qu'on met en suspension du DBCH de la modification II dans l'eau ou dans des substances organiques inertes et on le transforme quantitativement en modification V selon les revendications 1 à 6.

8. Médicament contenant le chlorhydrate cristallin suivant les revendications 1 à 6.

9. Utilisation du chlorhydrate cristallin suivant les revendications 1 à 5 pour la préparation de médicaments.
